# EUROPEAN PATENT SPECIFICATION

(11) **EP 2 792 304 B1**
(45) Date of publication and mention of the grant of the patent: **08.04.2020**
(21) Application number: 12858004.0
(22) Date of filing: 14.12.2012
(51) Int. Cl.: A61B 6/04, A61G 13/10

(54) **MEDICAL EXAMINATION-CUM-SURGERY TABLE**
TISCH FÜR MEDIZINISCHE UNTERSUCHUNGEN UND OPERATIONEN
TABLE D'EXAMEN ET D'OPÉRATION

(30) Priority: 16.12.2011 JP 2011276378
(43) Date of publication of application: 22.10.2014
(73) Proprietor: MIZUHO Corporation, Tokyo 113-0033 (JP)
(72) Inventor: OBI, Takuya, Bunkyo-ku Tokyo 113-0033 (JP)
(74) Representative: Viering, Jentschura & Partner mbB Patent- und Rechtsanwälte
(86) International application number: PCT/JP2012/082470
(87) International publication number: WO 2013/089220

(56) References cited:
- JP-A- 2005 152 089
- JP-A- 2007 105 346
- JP-A- 2007 136 201
- US-A- 5 596 779
- US-A1- 2005 129 181
- US-B1- 6 769 145

## Description

### Technical Field

The present invention relates to a medical treatment and surgical operation table, and more specifically, to a table commonly usable for medial treatment and surgical operation in which the table is freely movable in a horizontal direction and freely inclinable.

### Background Technology

In a known medical technical field, a known surgical operation table and a known medical treatment table have different functions from each other because of difference in their use. For example, it is required for a medical treatment table used for an X-ray apparatus as one example to be provided with a function such that a doctor can optionally move a table, in a horizontal direction, on which a patient is laid, for observation of an imaged portion with the X-ray transmitting a specified part of a body of the patient (see Patent Document 1).

On the other hand, it is necessary for a surgical operation table to be movable to a position at which a doctor can perform surgical treatment to a specific part of a patient body (i.e., it is necessary to change the position (posture) of the patient, and because of such reason, it is required for the table on which the patient is laid to be provided with a function of being vertically movable or inclinable (see Patent Document 2).

### Prior Art Document

### Patent Document

Patent Document 1: Japanese Patent Laid-open Publication No. 2010-99162
Patent Document 2: Japanese Patent Laid-open Publication No. 2004-73616
US-A-2005/129181 discloses a medical treatment and surgical operation table, which is freely movable in two horizontal directions, and freely inclinable. A first fixing/releasing means, which inhibits movement, when the table is in a horizontal position, is provided and comprises a permanent magnet and a coil, and a second fixing/releasing means, which inhibits lateral movement, as long as the table is tilted, is provided and comprises a tooth clutch.

### Disclosure of The Invention

### Problems to be solved by The Invention

Further, for example, in an occasion of inserting a catheter in a blood vessel in an endovascular treatment, there may cause a case when an emergent surgical operation is needed as occasion demands while photographing an X-ray image of the inserting state, and in such occasion, it is necessary to change the position of a patient during the operation, and hence, necessary to move the patient from a medial treatment table to a surgical operation table, which causes a time loss, and moreover, excessive loads for a worker and a patient.

In addition, although a medical treatment and surgical operation table which can be applied to a case of photographing a patient with an X-ray has been required, since a general surgical operation tale is equipped with metal parts such as metal frame, which obstructs the transmission (penetration) of the X-ray, it becomes difficult to merely apply the medical treatment table as a table for undergoing the surgical operation, thus being inconvenient.

Then, in order to obviate such inconvenience, an object of the present invention is to provide a medical treatment and surgical operation table (medical treatment/ surgical operation table) functioning commonly as medical treatment table and surgical operation table.

### Means for solving The Problem

The medical treatment and surgical operation table (2), according to claim 1, provided to achieve the above object includes: a table (40) which is formed of a material through which an X-ray transmits and on which a patient lays; an operation unit (10) that supports a lower side of one end portion of the table and operates the table so as to elevate, incline and move in all direction in a horizontal plane; a base (5) that supports the operation unit; and fixing/releasing means (65) that fixes movement of the table or moves the fixed table by magnetic force.

According to this medical treatment and surgical operation table, a switching operation from a fixing operation for stopping the movement of the table to a releasing operation for moving the fixed table can be easily performed, and since the fixing operation and the releasing operation can be promptly performed, an operator can be easily handled. In addition, the table is provided with a function as the medical treatment table and a function as a surgical operation table, so that an emergent surgical operation after the medical treatment can be smoothly undergone.

The medical treatment and surgical operation table according to claim 2 in that of claim 1 further includes another fixing/ releasing means (75) that fixes movement of the table or moves the fixed table, the another fixing/ releasing means having a fixing force for fixing the movement of the table larger than that of the first mentioned fixing/ releasing means, wherein the movement of the table is fixed by the first mentioned fixing/ releasing means at a time when the table is in a horizontal state, and at a time when the table is in an inclined state, the movement of the table is fixed by the another fixing/releasing means.

According to this medical treatment table and surgical operation table, since in a state in which the table is largely inclined, the fixing/ releasing means having a large fixing force is operated, the table is superior in safeness.

Furthermore, in the medical treatment and surgical operation table according to claim 3 in that of claim 2, the first mentioned fixing/ releasing means has a reaction speed for fixing the movement of the table or moving the fixed table faster than that of the another fixing/releasing means.

According to this medical treatment table and surgical operation table, the table fixing operation and the table releasing operation can be promptly performed, thus being convenient for an operator.

Moreover, in the medical treatment and surgical operation table according to claim 4 in that of claim 1, a plurality of fixing/releasing means may be provided, in which in the table being in the horizontal state, the movement of the table is fixed by at least selected one of the plural fixing/ releasing means, and in the table being in the inclined state, the movement of the table is fixed by all the fixing/releasing means.

According to this medical treatment and surgical operation table, the table can be easily controlled with a different fixing force in the table horizontal state and the table inclining state.

### Effects of The Invention

Since the table of the present invention is provided with the function for operating as medical treatment table and surgical operation table, an operator can be easily handled and is hence available for an emergent surgical operation after the medical treatment.

### Brief Description of The Drawings

[Fig. 1] is a perspective view illustrating an outer configuration of one example of a medical treatment and surgical operation table.
[Fig. 2] is a diagram explaining an operation example of a table of the medical treatment and operation table in X- and Z-directions.
[Fig. 3] is a diagram explaining an inclination example of the table of the medical treatment and surgical operation table in the X-direction.
[Fig. 4] is a diagram explaining an operation example of the table of the medical treatment and surgical operation table in the Y-direction.
[Fig. 5] is a diagram explaining an inclination example of the table of the medical treatment/ surgical operation table in the Y-direction.
[Fig. 6] is a view showing a structure of an inclining mechanism of the table of the medical treatment and surgical operation table in the X-direction.
[Fig. 7] is a structural view illustrating an arrangement example of a moving mechanism of a table in a horizontally moving portion in Y-direction and a table fixing/releasing mechanism, in which Fig. 7(a) is a plan view and Fig. 7(b) is a sectional view taken along the line I-I of Fig. 7(a).
[Fig. 8] is a structural view illustrating example of arrangement of a moving mechanism for moving the table of the horizontally movable portion in an X-direction of a table fixing/ releasing mechanism, in which Fig. 8(a) is a plan view and Fig. 8(b) is a sectional view taken along the line II-II of Fig. 8(a).
[Fig. 9] is a schematic structural view illustrating a first fixing/ releasing mechanism.
[Fig. 10] is a schematic structural view illustrating a second fixing/releasing mechanism, in which Fig. 10(a) is an illustration showing a released state and Fig. 10(b) is an illustration showing a fixed state. Mode for embodying The Invention

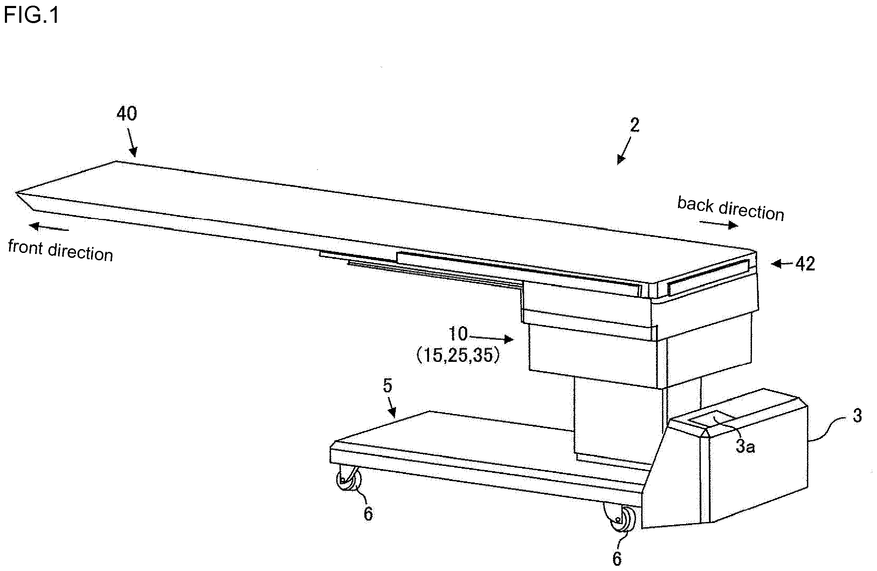

Hereunder, an embodiment for carrying out the present invention will be explained with reference to the accompanying drawings. For the sake of convenience, it is to be noted that, with reference to Fig. 1, a left-right direction is mentioned as an X-direction and a front-back direction of the medical treatment table, a vertical direction is mentioned as a Z-direction and a vertical direction of the medical treatment table, and a front/depth direction is mentioned as a Y-direction and a left-right direction of the medical treatment table. Further, the medical treatment table in the present embodiment is a table that is usable for a doctor in a field of the endovascular treatment using an X-ray apparatus. However, the present embodiment is not limited to such field and the table may be of course usable for a general surgical operation table or a general medical treatment table.

As shown in Fig. 1, a medical treatment and surgical operation table 2 of the present embodiment is provided with a base 5, a table operation unit 10 mounted on the base 5, a table 40 mounted on the table operation unit 10. Casters 6 may be equipped for lower four corners of the base 5 as occasion demands, and according to the attaching of these casters 6, the medical treatment and surgical operation table 2 is freely movable on a floor.

A case 3, in which a control device for electrically controlling the medical treatment and surgical operation table 2 and other members or like are accommodated, is mounted to the base 5. The case 3 is provided, on its surface, with a cover member 3a to be openable and closable, and a button or other parts, not shown, for electrically controlling the medical treatment and surgical operation table are optionally arranged inside the cover member 3a.

The table 40 is made of a carbon or like material capable of transmitting an X-ray and composed of one sheet plate-like member having a size by which a patient laying on the table can be supported. The table 40 is provided with a frame 42 covering the side portion of a rear end of the table so as to support the table 40 in the horizontal direction. The table 40 has a structure, except the rear end portion, not damaged by the transmission of the X-ray in its perpendicular direction. Further, for example, a head portion of a patient is placed on the front portion of the table 40 and a leg portion of the patient is placed on the rear portion thereof.

The table operation unit 10 is, as shown in Figs. 1 to 4, disposed on a lower portion of the table frame 42 and is provided with a plurality of operating mechanisms for performing various operations of the table 40. The operating mechanisms include an elevating mechanism 15 for vertically elevating the table 40, an inclining mechanism 25 for freely inclining the table 40, and a horizontally movable mechanism 35 for moving the table in the horizontal direction with hands.

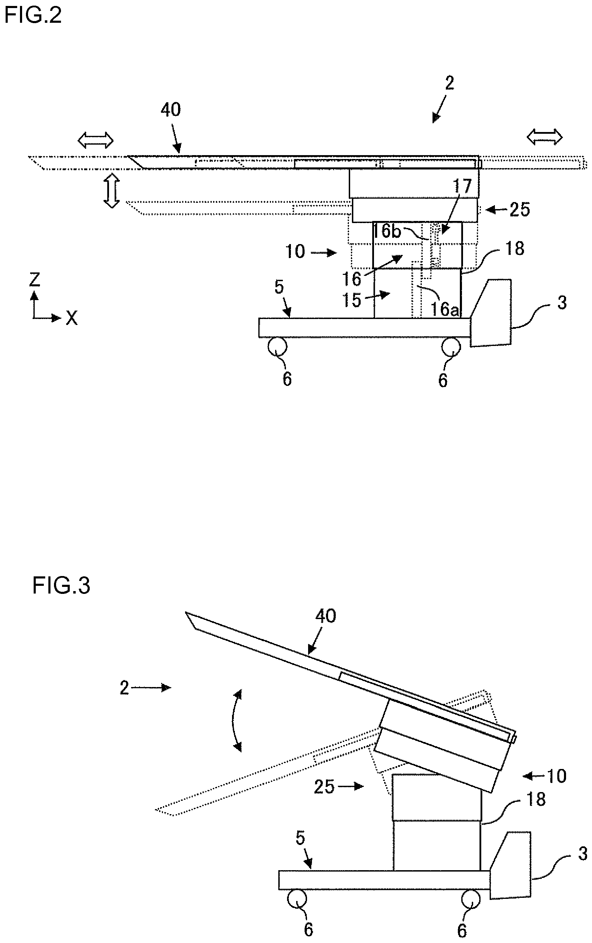

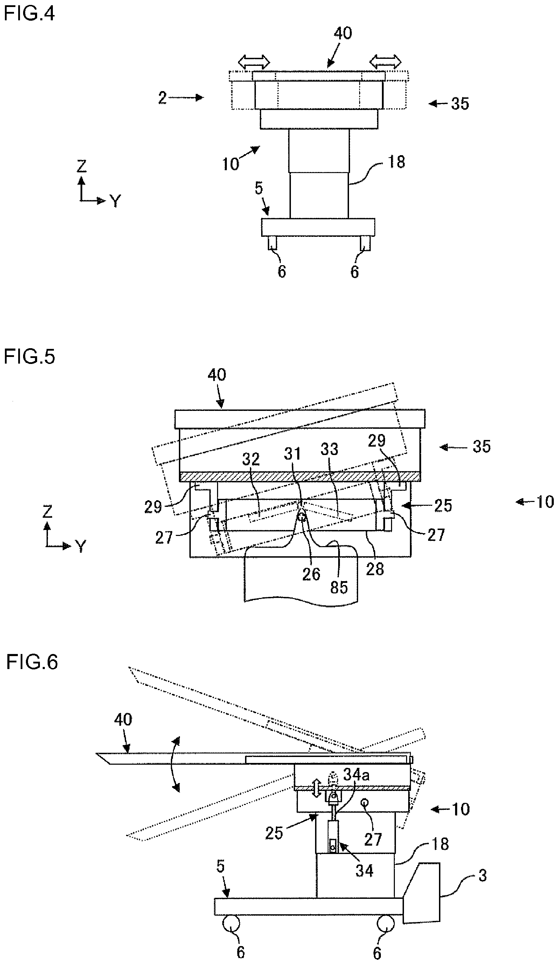

The elevating mechanism 15 is arranged, as shown in Fig. 2, above the base 5 to be expanded or contracted in the perpendicular direction, and as shown with arrow, to be adjustable in height level of the table in accordance with a height of a doctor. The inclining mechanism 25 is positioned above the elevating mechanism 15 and coupled with the table via a laterally turning shaft 26 and a longitudinally turning shaft 27 in a manner such that the table 40 can be laterally turned with the laterally turning shaft 26 being fulcrum while longitudinally turned with the longitudinally turning shaft 27 being fulcrum as shown in Fig. 6. Thus, the inclination of the table 40 can be adjusted by a doctor to thereby change a posture of the patient at which the doctor can easily treat the patient. Furthermore, the horizontally movable mechanism 35 is positioned, as shown in Figs. 2, 4, 7 and 8, above the inclining mechanism 25 so as to freely slide the table with hands in the longitudinally and laterally horizontal direction to thereby move the position of the patient, i.e., table 40, in the horizontally all directions thereof.

The elevating mechanism 15 is provided, as shown in Fig. 2 with dotted line, with a rod member 16 including a plurality of rods 16a and 16b combined to be expanded and contracted in the perpendicular direction between the base 5 and the inclining mechanism 25, and a hydraulic cylinder assembly 17 which slides the rod 16b in the vertical direction with respect to the rods 16a. The rod member 16 and the hydraulic cylinder assembly 17 are entirely covered by a vertically expandable cover 18. When the hydraulic cylinder assembly 17 is driven, the rod member 16 combined with the rods 16a and 16b is expanded or contracted to thereby vertically elevate the table 40 to a desired height level.

The inclining mechanism 25 is arranged, as shown in Figs. 5 and 6, between the elevating mechanism 15 and the horizontally movable mechanism 35 and provided with a housing 28 by which the laterally turning shaft 26 and the longitudinally turning shaft 27 are held. The housing 28 has a box-shaped structure substantially in form of rectangular parallelepiped shape, and is coupled with a bracket 85 upwardly standing from an upper end of the elevating mechanism 15 via the laterally turning shaft 26. On the other hand, the longitudinally turning shaft 27 is provided on each side of the horizontally arranged housing 28 perpendicular to the laterally turning shaft 26 so as to project outward in the horizontal direction, and the tip end of the longitudinally turning shafts 27 are coupled with left and right (lateral) downwardly directed brackets 29 projecting perpendicularly from the lower end of the horizontally movable mechanism 35.

Furthermore, a pin 31 is provided at a perpendicularly upper portion of the laterally turning shaft 26 in a manner held by the housing 28, and a rod is connected to the pin 31 provided with hydraulic cylinder assemblies 32 and 33 in laterally symmetric arrangement with respect to the laterally turning shaft 26. When cylinders of the respective cylinder assemblies 32 and 33 are reciprocated in directions reverse to each other, the rod pushes the pin 31 to thereby incline the table 40 in the lateral direction with a predetermined inclination with the laterally turning shaft being the fulcrum.

Further, as shown in Fig. 6, in front of the longitudinally turning shaft 27, a hydraulic cylinder assembly 34 is arranged so as to expand or contract a rod 34a in the perpendicular direction between the elevating mechanism 15 and the horizontally movable mechanism 35. When the hydraulic cylinder assembly 34 is driven, the length of the rod 34a is expanded or contracted so as to incline the front and rear portions of the table 40 at the predetermined inclination with the longitudinally turning shaft 27 being the fulcrum.

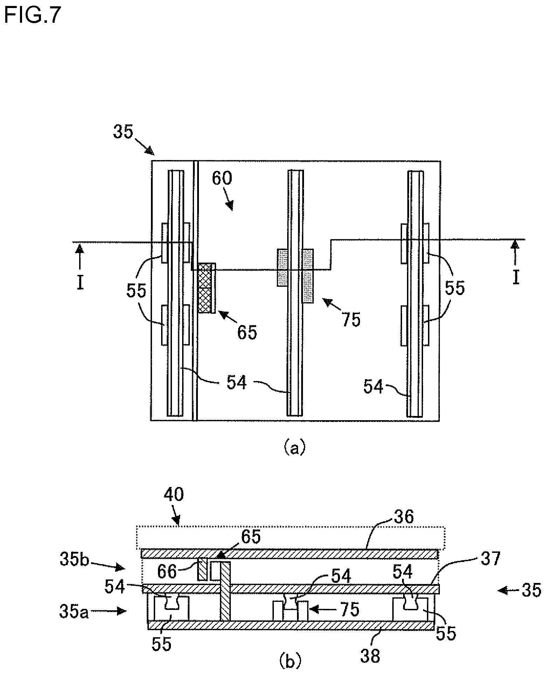

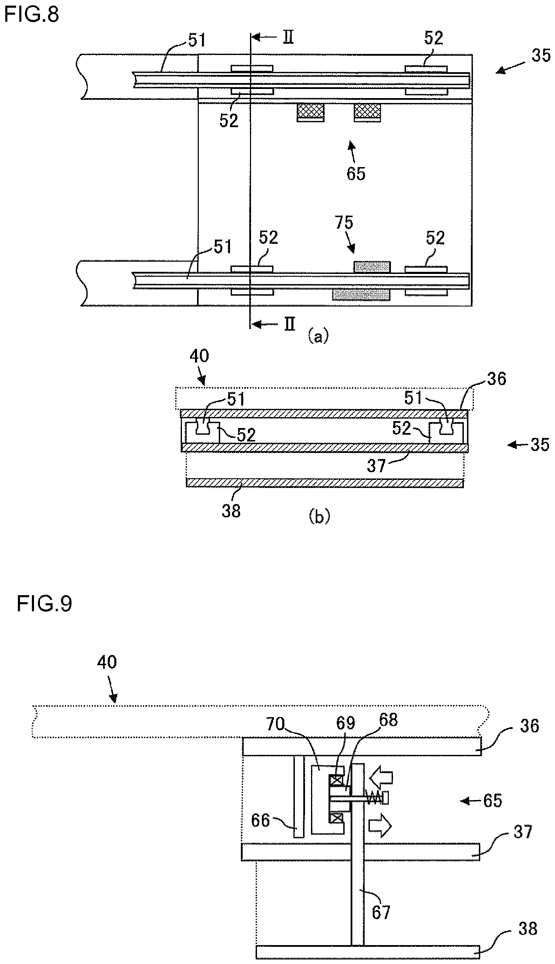

Furthermore, as shown in Figs. 7 and 8, the horizontally movable mechanism 35 is provided with a lower side housing 35a arranged above the inclining mechanism 25 and an upper side housing 35b attached to an upper end portion of the lower side housing to be slidable and coupled with the table 40. Further, an upper support member 36, a central support member 37 and a lower support member 38 are provided respectively with the upper end portion of the upper side hosing 35b, a boundary portion between the upper side housing 35b and the lower side housing 35a, and the lower end portion of the lower side housing 35a with predetermined intervals, respectively, in the horizontal direction.

As shown in Fig. 8, two vertical guide rails 51 arranged in parallel are attached to the upper support member (bracket) 36 so as to extend in the X-direction on both lateral side end portions within the upper side housing 35b. A plurality of guide rail support members 52 supporting the vertical guide rails 51 to be slidable on both lateral sides are mounted with equal interval to the center support member 37. The guide rails 51 are freely movable in the X-direction, and according to the movement of the guide rails 51, the upper support member 36 and the table 40 becomes movable in the front-and-rear (longitudinal) direction.

On the other hand, as shown in Fig. 7, three horizontal guide rails 54 arranged in parallel with equal interval are attached to the center support member 37 so as to extend in the lateral direction within the lower side housing 35a. A plurality of guide rail support members 55 supporting the horizontal guide rails 54 to be slidable on both lateral sides are mounted with equal interval to the lower support member 38. The guide rails 54 are freely movable in the Y-direction, and according to the movement of the guide rails 51, the upper support member 36 and the table 40 becomes movable in the lateral direction.

According to the horizontally movable mechanism 35 having the structure mentioned above, when an operator manually handles the table 40 in the longitudinal or lateral direction, the vertical guide rails 51 or horizontal guide rails 54 are moved within the respective support members 52, 55 for these guide rails to thereby freely move the table 40 in all the directions in the horizontal plane.

Furthermore, the medical treatment and surgical operation table 2 according to the present embodiment is provided with, as shown in Fig. 7(a), a fixing/releasing mechanism 60 for fixing the table 40 that is movable in the horizontal direction and releasing such fixing of the table for allowing the table to be movable. The fixing/releasing mechanism 60 includes a first fixing/releasing mechanism 65 and a second fixing/releasing mechanism 75 having different fixing force for fixing the movement of the table by the operator, in which the first fixing/ releasing mechanism 65 has a fixing force larger than that of the second fixing/releasing mechanism 75 and has a fixing/releasing time (i.e., speed) slower than that of the second fixing/ releasing mechanism.

More specifically, the first fixing/releasing mechanism 65 is one for fixing the movement of the table by utilizing magnetic force, and as shown in Fig. 9, includes an iron plate 66 attached to the upper support member 36 and having a magnetic force, a permanent magnet 68 pushed against a support member 67 attached to the lower support member 38 with a spring force, a coil 69 arranged around the permanent magnet, and a cover member 70 arranged apart from the iron plate so as to cover the permanent magnet 68 and the coil 69. The movement of the table 40 is fixed by adsorbing the permanent magnet 68 (cover member 70) arranged apart from the iron plate 66. Further, on the other hand, by applying current to the coil 69, the magnetic force of the permanent magnet 68 is neutralized and the permanent magnet 68 is separated from the iron plate 66 to thereby release the fixing of the table to be movable.

Further, although the first fixing/releasing mechanism 65 is merely composed of a permanent magnet and a coil, it may be composed of a mechanism generally called an electromagnet.

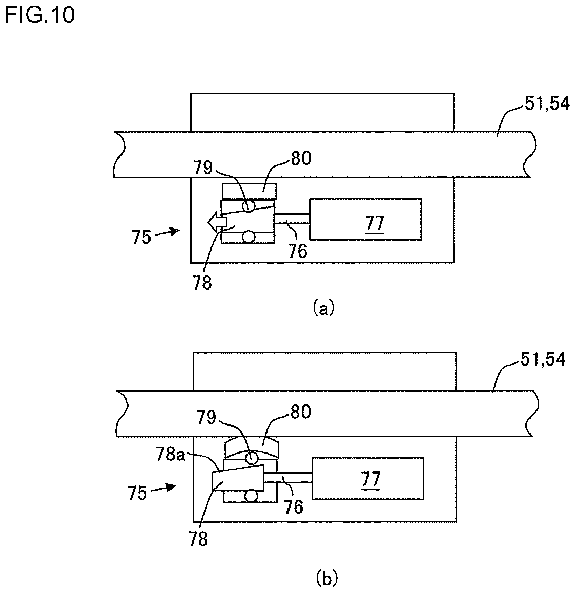

On the other hand, the second fixing/releasing mechanism 75 includes, as shown in Fig. 10, a motor 77 including a rotating shaft 76, a cam 78 that is moved by the rotation of the rotating shaft 76, a pin 79 engaging the cam 78, and a push member 80 for pushing at least one side surface of the guide rails 51 and 54 arranged between the cam 78 and the guide rails 51 and 54. According to such structure, when the motor is driven, the rotating shaft 76 of the motor 77 is expanded or contracted to thereby move the cam 78, and hence, the push member 80 pushes the one side surface of the guide rails 51, 54 by the pin 79 engaged with the inclination surface 78a formed to the cam 78, thereby fixing the movement of the guide rails 51 and 54. On the other hand, since the cam 78 is moved to the original position by reverse rotation of the motor , the push member is separated from the side surfaces of the guide rails 51 and 54, so that the fixing of the guide rails 51 and 54 is released to be movable.

Further, when the table 40 is moved in the horizontal direction, it is required for the table 40 to be promptly fixed and/or for the fixed table to be promptly moved, and when the table 40 is inclined, it is required for the table to have high fixing force so as not to be moved. Therefore, in the present embodiment, it is desirable to use the first fixing/releasing mechanism 65 as fixing/releasing means at the time of moving the table 40 in the horizontal direction, and also use the second fixing/ releasing mechanism 75 as table fixing means at the time of inclining the table 40.

Furthermore, as the second fixing/releasing mechanism, a hydraulic mechanism or pneumatic mechanism may be used instead of the motor so as to bring the push member 80 into contact with the guide rails 51 and 54.

Next, an example of using the medical treatment and surgical operation table will be explained, and herein, the first and second fixing/releasing mechanisms 65 and 75 are controlled by a button (buttons) for releasing, not shown, in which the first fixing/ releasing mechanism 65 is always fixed and released only at a time of operating the button for releasing. Further, the second fixing/releasing mechanism 75 of the present embodiment is controlled so that it is released at a time when the table 40 takes the horizontal position and automatically fixed only at a time when the table 40 is inclined. Moreover, the elevation and/or inclination control of the table 40 is performed by operating a predetermined button for elevation and/or inclination.

In the operation at an initial stage, as shown in Fig. 1, the table 40 is held to be horizontally. Then, when an operator operates a button for releasing, the table 40 becomes movable in the lateral and longitudinal directions to be moved in all the directions by the movement of the guide rails 51 and 54. On the contrary, when the operation of the button for releasing is stopped, the movement of the guide rails 51 and 54 fixed by the first fixing/releasing mechanism 65 is fixed, and hence, the table 40 becomes immovable.

Further, when it is required to further move the table 40, the fixing of the table 40 (i.e., fixing of the guide rails 51, 54) can be released by operating the button for releasing. Although the fixing force of the first fixing/releasing mechanism is weak, since the response is prompt, the fixing or releasing of the table 40 can be promptly performed, thus being convenient for the operator in use.

Furthermore, in an occasion of using the table as surgical operation table, when the table is elevated or inclined, the elevation or inclination thereof can be done by operating a button for inclination. In the inclining position of the table 40, the movement of the guide rails 51 and 54 is fixed for the sake of safety so that the table 40 is not automatically moved by the second fixing/releasing mechanism 75. In this time, the second fixing/releasing mechanism 75 is controlled so as not to be released even if the button for releasing is operated.

Although the second fixing/releasing mechanism 75 has the reaction promptness is slower than that of the first fixing/releasing mechanism 65, since it has the fixing force higher than that of the first one, the table 40 is not moved during the inclining operation, thus being safeness.

As mentioned hereinabove, the medical treatment and surgical operation table of the present embodiment is provided with two fixing/releasing mechanisms having different fixing forces and reaction speeds for fixing or releasing the movement of the table 40 in the horizontal direction so as to be operated separately in accordance with requirement for use, and accordingly, the medical treatment and surgical operation table can be conveniently handled for the operator in use.

It is further to be noted that the present embodiment is one example and the present invention is not limited thereto. For example, the table operation mechanisms of the present embodiment may be replaced with other generally known mechanisms. Moreover, even for the first fixing/releasing mechanism, it is important to includes with a plurality of fixing/releasing mechanisms having different fixing forces and reaction speeds, and hence, it may be possible to use other electric or mechanical mechanisms in place thereof. Furthermore, a plurality of fixing/releasing mechanisms having same fixing forces and reaction speeds may be provided, and in this case, only in a time when the table is horizontally moved, selective ones of the plural fixing/releasing mechanisms are operated. More specifically, for example, at a time when the horizontal movement of the table is fixed or released, the movement of the table is fixed by one or two fixing/releasing mechanisms of the plural ones, and on the other hand, at a time when the movement of the table in the inclining time is fixed or released, the movement of the table is fixed by all the fixing/releasing mechanisms. According to such operation manner, the fixing force can be controlled in different manners in the horizontal state and inclination state of the table 40.

### Reference Numerals

2 --- medical treatment and surgical operation table
5 --- base
10 --- operation unit
40 --- table
65 --- first fixing/ releasing mechanism (fixing/ releasing means)
75 --- second fixing/releasing mechanism (another fixing/releasing means)

## Claims

1. A medical treatment and surgical operation table (2) comprising:
a table (40) which is formed of a material through which an X-ray transmits and on which a patient lays;
an operation unit (10) that supports a lower side of one end portion of the table (40) and includes: an elevating mechanism (15) for vertically elevating the table (40), an inclining mechanism (25) for freely inclining the table (40), and a horizontally movable mechanism (35) for moving the table (40) in the horizontal direction, wherein the horizontally movable mechanism (35) comprises vertical guide rails (51) slidably supported by corresponding vertical guide rail support members (52), and horizontal guide rails (54) slidably supported by corresponding horizontal guide rail support members (54);
a base (5) that supports the operation unit (10); and
fixing/releasing means (60) configured to fix a movement of the table (40) or to release the fixing of the table (40), the fixing/releasing means (60) comprising a first fixing/releasing mechanism (65) and a second fixing/releasing mechanism (75),
wherein the first fixing/releasing mechanism (65) comprises a permanent magnet (68) and a coil (69) arranged around the permanent magnet (68), wherein by applying a current to the coil (69), the magnetic force of the permanent magnet (68) is neutralized, thereby releasing the fixing of the table (40) to be movable, wherein the second fixing/releasing mechanism (75) comprises a motor (77) including a rotating shaft (76), a cam (78) movable by a rotation of the rotating shaft (76), a pin (79) engaged with an inclination surface (78a) of the cam (78), and a push member (80) for pushing at least one side surface of the guide rails (51, 54), wherein when the motor (77) is driven, the rotating shaft (76) of the motor (77) is expanded to thereby move the cam (78) and to thereby push the push member (80) against one side surface of the guide rails (51, 54) by the pin (79), thereby fixing the movement of the guide rails (51, 54), and when the cam (78) is moved to the original position by reverse rotation of the motor (77), the push member (80) is separated from the side surface of the guide rails (51, 54), thereby releasing the fixing of the table (40),
wherein the second fixing/releasing mechanism (75) has a fixing force for fixing the movement of the table (40) that is larger than that of the first fixing/releasing mechanism (65),
wherein when the table (40) is in a horizontal position, the table (40) is held fixed by the first fixing/releasing mechanism (65) and is released only at a time of operating a button for releasing, wherein the second fixing/releasing mechanism (75) is controlled so that it is released at a time when the table (40) takes the horizontal position and is automatically fixed only at a time when the table (40) is inclined.

2. The medical treatment and surgical operation table (2) according to claim 1, wherein the first fixing/releasing mechanism (65) has a reaction speed for fixing the movement of the table (40) or releasing the fixing of the table (40) faster than that of the second fixing/releasing mechanism (75).

## Patentansprüche

1. Tisch (2) für medizinische Behandlung und chirurgische Eingriffe, aufweisend:
einen Tisch (40), der aus einem für Röntgenstrahlen durchlässigen Material hergestellt ist und auf welchem ein Patient liegt,
eine Betätigungseinheit (10), die eine untere Seite eines Endabschnitts des Tisches (40) trägt und aufweist: einen Hebemechanismus (15) zum vertikalen Anheben des Tisches (40), einen Neigungsmechanismus (25) zum freien Neigen des Tisches (40) und einen horizontal bewegbaren Mechanismus (35) zum Bewegen des Tisches (40) in der horizontalen Richtung, wobei der horizontal bewegbare Mechanismus (35) aufweist: vertikale Führungsschienen (51), die von korrespondierenden Vertikale-Führungsschiene-Trageelementen (52) gleitend getragen sind, und horizontale Führungsschienen (54), die von korrespondierenden Horizontale-Führungsschiene-Trageelementen (54) gleitend getragen sind,
eine Basis (5), die die Betätigungseinheit (10) trägt, und
Festlegungs-/Freigabemittel (60), die dazu eingerichtet sind, eine Bewegung des Tisches (40) festzulegen oder die Festlegung des Tisches (40) freizugeben, wobei die Festlegungs-/Freigabemittel (60) einen ersten Festlegungs-/Freigabemechanismus (65) und einen zweiten Festlegungs-/Freigabemechanismus (75) aufweisen,
wobei der erste Festlegungs-/Freigabemechanismus (65) einen Permanentmagneten (68) und eine um den Permanentmagneten (68) herum angeordnete Spule (69) aufweist, wobei durch Anlegen eines Stromes an die Spule (69) die magnetische Kraft des Permanentmagneten (68) neutralisiert wird, wodurch die Festlegung des Tisches (40) freigegeben wird, um bewegbar zu sein, wobei der zweite Festlegungs-/Freigabemechanismus (75) aufweist: einen eine Drehwelle (76) aufweisenden Motor (77), einen Nocken (78), der durch eine Drehung der Drehwelle (76) bewegbar ist, einen Stift (79), der mit einer Neigungsfläche (78a) des Nockens (78) in Eingriff steht, und ein Drückelement (80) zum Drücken wenigstens einer Seitenfläche der Führungsschienen (51, 54), wobei, wenn der Motor (77) angetrieben wird, die Drehwelle (76) des Motors (77) ausgefahren wird, um dadurch den Nocken (78) zu bewegen und um dadurch das Drückelement (80) gegen eine Seitenfläche der Führungsschienen (51, 54) durch den Stift (79) zu drücken, wodurch die Bewegung der Führungsschienen (51, 54) festgelegt wird, und, wenn der Nocken (78) zu der ursprünglichen Position durch eine umgekehrte Drehung des Motors (77) bewegt wird, das Drückelement (80) von der Seitenfläche der Führungsschienen (51, 54) getrennt wird, wodurch die Festlegung des Tisches (40) freigegeben wird,
wobei der zweite Festlegungs-/Freigabemechanismus (75) eine Festlegungskraft zum Festlegen der Bewegung des Tisches (40) aufweist, die größer als die des ersten Festlegungs-/Freigabemechanismus (65) ist,
wobei, wenn sich der Tisch (40) in einer horizontalen Position befindet, der Tisch (40) durch den ersten Festlegungs-/Freigabemechanismus (65) festgelegt gehalten wird und nur zu einem Zeitpunkt freigegeben wird, zu dem ein Freigabeknopf betätigt wird, wobei der zweite Festlegungs-/Freigabemechanismus (75) derart gesteuert wird, dass er zu einem Zeitpunkt freigegeben wird, wenn der Tisch (40) die horizontale Position einnimmt, und nur zu einem Zeitpunkt automatisch festgelegt wird, wenn der Tisch (40) geneigt ist.

2. Tisch (2) für medizinische Behandlung und chirurgische Eingriffe nach Anspruch 1, wobei der erste Festlegungs-/Freigabemechanismus (65) eine Reaktionsgeschwindigkeit zum Festlegen der Bewegung des Tisches (40) oder zum Freigeben der Festlegung des Tisches (40) aufweist, die höher als diejenige des zweiten Festlegungs-/Freigabemechanismus (75) ist.

## Revendications

1. Table de traitement médical et d'opération (2) comprenant :
une table (40) qui est formée avec un matériau à travers lequel un rayon X est transmis et sur laquelle un patient est allongé ;
une unité opérationnelle (10) qui supporte un côté inférieur d'une partie d'extrémité de la table (40) et comprend : un mécanisme de levage (15) pour lever verticalement la table (40), un mécanisme d'inclinaison (25) pour incliner librement la table (40) et un mécanisme horizontalement mobile (35) pour déplacer la table (40) dans la direction horizontale, dans laquelle le mécanisme horizontalement mobile (35) comprend des rails de guidage verticaux (51) supportés de manière coulissante par des éléments de support de rail de guidage verticaux (52) correspondants et des rails de guidage horizontaux (54) supportés de manière coulissante par des éléments de support de rail de guidage horizontaux (54) correspondants ;
une base (5) qui supporte l'unité opérationnelle (10) ; et
un moyen de fixation / libération (60) configuré pour fixer un mouvement de la table (40) ou pour libérer la fixation de la table (40), le moyen de fixation / libération (60) comprenant un premier mécanisme de fixation / libération (65) et un second mécanisme de fixation / libération (75),
dans laquelle le premier mécanisme de fixation / libération (65) comprend un aimant permanent (68) et une bobine (69) agencée autour de l'aimant permanent (68), dans laquelle, en appliquant un courant sur la bobine (69), la force magnétique de l'aimant permanent (68) est neutralisée, libérant ainsi la fixation de la table (40) pour qu'elle soit mobile, dans laquelle le second mécanisme de fixation / libération (75) comprend un moteur (77) comprenant un arbre de rotation (76), une came (78) mobile par une rotation de l'arbre de rotation (76), une broche (79) mise en prise avec une surface d'inclinaison (78a) de la came (78), et un élément de poussée (80) pour pousser au moins une surface latérale des rails de guidage (51, 54), dans laquelle lorsque le moteur (77) est entraîné, l'arbre de rotation (76) du moteur (77) est expansé afin de déplacer ainsi la came (78) et afin de pousser ainsi l'élément de poussée (80) contre une surface latérale des rails de guidage (51, 54) par la broche (79), fixant ainsi le mouvement des rails de guidage (51, 54), et lorsque la came (78) est déplacée dans la position d'origine par la rotation inverse du moteur (77), l'élément de poussée (80) est séparé de la surface latérale des rails de guidage (51, 54), libérant ainsi la fixation de la table (40),
dans laquelle le second mécanisme de fixation / libération (75) a une force de fixation pour fixer le mouvement de la table (40) qui est supérieure à celle du premier mécanisme de fixation / libération (65),
dans laquelle lorsque la table (40) est dans une position horizontale, la table (40) est maintenue fixée par le premier mécanisme de fixation / libération (65) et est libérée uniquement au moment de l'actionnement d'un bouton pour la libération, dans laquelle le second mécanisme de fixation / libération (75) est contrôlé de sorte qu'il est libéré à un moment où la table (40) adopte la position horizontale et est automatiquement fixée uniquement au moment où la table (40) est inclinée.

2. Table de traitement médical et d'opération (2) selon la revendication 1, dans laquelle le premier mécanisme de fixation / libération (65) a une vitesse de réaction pour fixer le mouvement de la table (40) ou libérer la fixation de la table (40), plus rapide que celle du second mécanisme de fixation / libération (75).
